# EUROPÄISCHE PATENTSCHRIFT

(11) **EP 2 962 525 B1**
(45) Veröffentlichungstag und Bekanntmachung des Hinweises auf die Patenterteilung: **01.02.2017**
(21) Anmeldenummer: 14706647.6
(22) Anmeldetag: 26.02.2014
(51) Int. Cl.: H05B 3/34

(54) **HEIZELEMENT MIT FLÄCHIGER, WÄRME ENTWICKELNDER SCHICHT**
HEATING ELEMENT HAVING A PLANAR, HEAT-GENERATING LAYER
ÉLÉMENT CHAUFFANT COMPRENANT UNE COUCHE THERMOGÉNÉRATRICE PLANE

(30) Priorität: 01.03.2013 DE 102013203584
(43) Veröffentlichungstag der Anmeldung: 06.01.2016
(73) Patentinhaber: Fraunhofer-Gesellschaft zur Förderung der angewandten Forschung e.V., 80686 München (DE); Beiersdorf AG, 20253 Hamburg (DE)
(72) Erfinder: KÖNIG, Christian, 70794 Filderstadt (DE); NEMEC, Dominik, 70176 Stuttgart (DE); MAIER, Mathias, 70374 Stuttgart (DE); WÖLLER, Karl-Heinz, 20257 Hamburg (DE)
(74) Vertreter: Richardt Patentanwälte PartG mbB
(86) Internationale Anmeldenummer: PCT/EP2014/053764
(87) Internationale Veröffentlichungsnummer: WO 2014/131807

(56) Entgegenhaltungen:
- EP-A1- 1 988 748
- WO-A2-2008/091001
- DE-A1- 19 831 023
- DE-U1- 20 308 113
- JP-A- H11 214 131
- US-A1- 2010 258 334

## Beschreibung

Die Erfindung betrifft ein Heizelement mit einer flächigen, Wärme entwickelnden Schicht, ein Pflaster mit einem Heizelement und ein Verfahren zur Herstellung eines Heizelements.

Heizelemente mit flächigen, Wärme entwickelnden Schichten können eine vielfältige Anwendung finden. Anwendungen reichen von elektrischen Sitzheizungen für Fahrzeuge, Heizungen für textile Kleidung bis hin zu Anwendungen zur medizinischen und kosmetischen Behandlung von Mensch und Tier. Die WO 2010/028740 A1 offenbart zum Beispiel ein Wärmepflaster, aufweisend eine Wärme entwickelnde Schicht, wobei die Wärme entwickelnde Schicht einen Gehalt von Kohlenstoff-Nanoröhren aufweist. Die DE 20 2007 007 357 U1 offenbart einen Stoff in Form eines gewirkten, gewebten oder vliesähnlichen Kleidungsteils mit in diesem integrierten oder daran angebrachten Faserbündeln, die eine Vielzahl von Kunststofffilamenten aufweisen, welche auf ihrer Oberfläche eine leitfähige Beschichtung tragen.

Dokument US 2010/258334 A1 offenbart ein Heizelement mit einer flächigen wärmeentwickelnden Schicht und zwei elektrischen Kontaktierungen zur Bereitstellung eines elektrischen Heizstroms, wobei die elektrische Kontaktierungen an der wärmeentwickelnden Schicht an einander gegenüberliegenden Seiten der Schicht angeordnet sind.

Der Erfindung liegt die Aufgabe zugrunde, ein verbessertes Heizelement mit einer flächigen, Wärme entwickelnden Schicht zu schaffen. Die der Erfindung zugrunde liegende Aufgabe wird durch die Merkmale der unabhängigen Patentansprüche gelöst. Bevorzugte Ausführungsformen der Erfindung sind in den abhängigen Patentansprüchen angegeben.

Es wird ein Heizelement mit einer flächigen, Wärme entwickelnden Schicht und zwei elektrischen Kontaktierungen zur Bereitstellung eines elektrischen Heizstroms beschrieben, wobei die elektrische Kontaktierungen an der Wärme entwickelnde Schicht in paralleler Richtung zur Fläche der Schicht gesehen an einander gegenüberliegenden Seiten der Schicht angeordnet sind. Zumindest eine Kontaktierung umfasst ein erstes Kontaktelement und ein zweites Kontaktelement, wobei sich das erste Kontaktelement in der Wärme entwickelnden Schicht in einer ersten Ebene senkrecht zur Fläche der Schicht gesehen geschwungen erstreckt, wobei sich das zweite Kontaktelement parallel zur Erstreckungsrichtung des ersten Kontaktelements erstreckt. Das erste Kontaktelement und das zweite Kontaktelement weisen in der Erstreckungsrichtung gesehen eine Vielzahl von gemeinsamen Kontaktpunkten auf. Die elektrischen Kontaktierungen dienen hier als Elektroden, um eine Schnittstelle für den einzuspeisenden elektrischen Strom und die Wärme entwickelnde Schicht bereitzustellen.

Ausführungsformen der Erfindung könnten den Vorteil haben, dass eine optimale Zufuhr des elektrischen Heizstroms zur Gesamtheit der Wärme entwickelnden Schicht gewährleistet werden könnte. Selbst wenn also die Wärme entwickelnde Schicht den elektrischen Heizstrom nicht nur an ihrer Oberfläche, sondern innerhalb des Volumen-Bereichs selbst leitet, kann damit der gesamte Strom leitende Querschnitt der Wärme entwickelnden Schicht zur Wärmeentwicklung herangezogen werden.

Da sich hierzu das erste Kontaktelement in der Wärme entwickelnden Schicht geschwungen erstreckt, erfordert dies ein erstes Kontaktelement mit einer großen Länge. Insbesondere wenn das erste Kontaktelement einen höheren elektrischen Widerstand hat, kann durch die gleichzeitige Verwendung des zweiten Kontaktelements parallel zur Erstreckungsrichtung des ersten Kontaktelements und die regelmäßige Kontaktierung zwischen erstem und zweitem Kontaktelement an der Vielzahl von gemeinsamen Kontaktpunkten gewährleistet werden, dass sämtliche Bereiche der flächigen Wärme entwickelnden Schicht gleichmäßig mit dem elektrischen Heizstrom versorgt werden. Hierdurch könnte also insbesondere die Ausbildung eines sogenannten "Hotspots" vermieden werden, also eine lokale Temperaturüberhöhung aufgrund einer ungünstigen Zuleitung des elektrischen Heizstroms zu einzelnen Bereichen der Wärme entwickelnden Schicht. Dadurch könnte sich insgesamt eine homogene Erwärmung der Wärme entwickelnden Schicht ergeben.

Nach einer Ausführungsform der Erfindung erstreckt sich das zweite Kontaktelement in einer zweiten Ebene parallel zur Fläche der Schicht gesehen. Bevorzugter Weise befindet sich das zweite Kontaktelement auf der Oberfläche der Wärme entwickelnden Schicht und ist dort mittels geeigneter Befestigungsmittel mit der Oberfläche fixiert. Beispielsweise könnte das zweite Kontaktelement aufgeklebt sein. Ebenfalls möglich ist, dass das zweite Kontaktelement in der Wärme entwickelnden Schicht selbst enthalten ist. Wird die Wärme entwickelnde Schicht beispielsweise durch ein Spritzgussverfahren hergestellt, könnte das zweite Kontaktelement in die Wärme entwickelnde Schicht selbst eingegossen sein.

Das zweite Kontaktelement könnte ein gerader Leiter sein, welcher sich parallel zur Erstreckungsrichtung des ersten Kontaktelements erstreckt. Vorzugsweise erstreckt sich jedoch das zweite Kontaktelement in der zweiten Ebene geschwungen.

Es sei angemerkt, dass im Rahmen der gesamten Beschreibung als eine geschwungene Erstreckung des ersten und/oder zweiten Kontaktelements eine Erstreckung verstanden wird, deren Lage z.B. mäanderförmig, zigzagförmig, bogenförmig oder rechteckförmig in der jeweiligen ersten oder zweiten Ebene variiert. Nach einer Ausführungsform der Erfindung ist das erste Kontaktelement und/oder das zweite Kontaktelement in die Wärme entwickelnde Schicht durch eine bei textilen Fügeverfahren verwendbare Verbindungstechnik eingebracht. Unter textilem Fügen werden dabei allgemein Fertigungsverfahren verstanden, durch welche formschlüssig das erste Kontaktelement und/oder das zweite Kontaktelement mit der Wärme entwickelnden Schicht verbunden werden. Beispielsweise könnte das textile Fügen ein Einnähen, ein Einknüpfen, ein Einsticken oder ein Einweben der Kontaktelemente in die Wärme entwickelnde Schicht umfassen. Dadurch könnte sich insgesamt eine einfache und komfortable Möglichkeit ergeben, wie insbesondere in einem Massenproduktionsverfahren die flächige Wärme entwickelnde Schicht mit den elektrischen Kontaktierungen versehen werden kann. Die Kontaktierung ist dabei hochstabil, da im Gegensatz zu Kleben oder Aufpressen von Kontaktierungen auf die Wärme entwickelnde Schicht durch die formschlüssige Verbindung zwischen Kontaktelement und Wärme entwickelnder Schicht eine nahezu Irreversibilität gegenüber unbeabsichtigtem Lösen gewährleistet werden könnte.

Nach einer Ausführungsform der Erfindung weisen das erste Kontaktelement und das zweite Kontaktelement durch ineinander hängende Schlaufen die Vielzahl von gemeinsamen Kontaktpunkten auf. Dies könnte ermöglichen, dass in einem einfachen textilen Verbindungsverfahren "automatisch" eine regelmäßige Kontaktierung zwischen dem ersten Kontaktelement und dem zweiten Kontaktelement hergestellt werden könnte. Die Schlaufen ergeben sich beispielsweise daraus, dass sich ja das erste Kontaktelement bereits geschwungen in der ersten Ebene erstreckt. Die hieraus resultierenden Schlaufen könnten dann in gegebenenfalls vorhandene Schlaufen des zweiten Kontaktelements eingreifen oder aber im Falle eines geraden zweiten Kontaktelements direkt um das zweite Kontaktelement greifen.

Nach einer Ausführungsform der Erfindung durchdringt das erste Kontaktelement die Wärme entwickelnde Schicht in Richtung senkrecht zur Fläche der Wärme entwickelnden Schicht vollständig. Dadurch könnte gewährleistet sein, dass der gesamte Querschnitt der Wärme entwickelnden Schicht zur Kontaktierung zwischen den Kontaktelementen herangezogen wird. Der elektrische Strom wird also über den gesamten Querschnitt der Wärme entwickelnden Schicht bereitgestellt.

Nach einer Ausführungsform der Erfindung umfasst das erste Kontaktelement ein elektrisch leitfähiges Garn. Beispielsweise könnte es sich hierbei um mit Silber beschichtetes Nylon handeln. Durch das insbesondere Hindurchstecken eines Garns durch die Wärme entwickelnde Schicht ergibt sich eine mechanisch stabile, formschlüssige Verbindung, wobei eine gute mechanische Anbindung garantiert werden kann. Dies führt zu geringen elektrischen Übergangswiderständen zwischen Kontaktelement und Wärme entwickelnder Schicht. Durch die Flexibilität eines solchen Garns ist zudem gewährleistet, dass sich das Kontaktelement zusammen mit der Wärme entwickelnden Schicht verformen kann. Bewegt sich also die Wärme entwickelnde Schicht, kann das elektrisch leitfähige Garn problemlos dieser Bewegung folgen. Da jedoch das elektrisch leitfähige Garn unter Umständen einen erhöhten elektrischen Widerstand haben könnte als beispielsweise eine Kupferlitze, ergibt sich bei der Verwendung eines elektrisch leitfähigen Garns zusammen mit dem zweiten Kontaktelement der Vorteil, dass über die Vielzahl von Kontaktpunkten dennoch die gesamte Wärme entwickelnde Schicht gleichmäßig mit elektrischem Strom versorgt werden kann. Nichtsdestotrotz ist es möglich, in einfacher Art und Weise das erste Kontaktelement zum Beispiel in Nähprozessen zu verarbeiten.

Zum Beispiel handelt es sich bei dem ersten Kontaktelement um einen Oberfaden und bei dem zweiten Kontaktelement um einen Unterfaden. Bei Betrachtung einer einfachen Nähmaschine könnte damit das elektrisch leitfähige Garn als Oberfaden zum Einsatz kommen und beispielsweise ein metallischer Draht oder ebenfalls ein weiteres elektrisches leitfähiges Garn als Unterfaden. Allgemein ist jedoch bevorzugt, dass das zweite Kontaktelement einen metallischen Draht umfasst, beispielsweise eine Kupferlitze. Dies gewährleistet eine hohe elektrische Leitfähigkeit und damit einen geringen elektrischen Widerstand und damit eine optimale Stromverteilung zum ersten Kontaktelement.

Nach einer weiteren Ausführungsform der Erfindung ist die Wärme entwickelnde Schicht aufgrund einer Dehnbewegung elastisch dehnbar, wobei sich das erste Kontaktelement in der ersten Ebene mit einer räumlichen Schwingungsperiode geschwungen erstreckt, wobei das erste Kontaktelement dazu ausgebildet ist, aufgrund einer Änderung der Schwingungsperiode der elastischen Dehnbewegung zu folgen. Dies bedeutet, dass das erste Kontaktelement nicht so in einer Art und Weise mit der Wärme entwickelnden Schicht verbunden ist, dass die Wärme entwickelnde Schicht gegen jegliche Art von Dehnbewegung durch das erste Kontaktelement gesichert ist. Gegenteilig sollte das erste Kontaktelement so mit der Wärme entwickelnden Schicht in Kontakt stehen, dass bei einer Bewegung, insbesondere einer Dehnbewegung der Wärme entwickelnden Schicht, dieses erste Kontaktelement durch eine vorzugsweise nicht Längen verändernde Formveränderung dieser Dehnbewegung folgen kann.

Im Rahmen der vorliegenden Beschreibung wird unter einer räumlichen Schwingungsperiode das kleinste örtliche Intervall verstanden, nach dem sich der Vorgang des räumlichen Schwingungsverlaufs des ersten Kontaktelements wiederholt.

Es sei angemerkt, dass durchaus das erste Kontaktelement und/oder das zweite Kontaktelement auch zusätzlich elastisch dehnbar ausgestaltet sein kann.

Nach einer weiteren Ausführungsform der Erfindung weist die flächige Wärme entwickelnde Schicht in ihrem Volumen homogen verteilt elektrisch leitfähige Nanoröhren und/oder ein elektrisch leitfähiges Polymer auf. Im Vergleich beispielsweise zur Verwendung von Heizdrähten ergibt sich damit homogen über die gesamte Fläche eine gleichmäßige Wärmeentwicklung der Wärme entwickelnden Schicht, wohingegen im Falle von Heizdrähten oder gewobenen Heizmatten eine erhöhte Wärmeentwicklung am exakten Ort der verwendeten Heizfilamente gegeben ist. Durch die flächige Wärmeentwicklung wird jedoch der Entstehung von unerwünschten Hotspots vorgebeugt.

Es sei an dieser Stelle angemerkt, dass insbesondere im Falle der Verwendung von elektrisch leitfähigen Nanoröhren das Phänomen auftauchen könnte, dass die Nanoröhren aufgrund des Herstellungsvorgangs, beispielsweise eines Spritzgussvorgangs, an der Oberfläche der Wärme entwickelnden Schicht ausgerichtet werden. Dies resultiert aus einer sogenannten "Spritzhaut" an der Oberfläche, welche jedoch eine geringere elektrische Leitfähigkeit aufweist als das Innere der flächigen Wärme entwickelnden Schicht. Aus diesem Grund eignet sich hier in besonderer Weise die beschriebene geschwungene Erstreckung des ersten Kontaktelements in der Wärme entwickelnden Schicht in der ersten Ebene senkrecht zur Fläche der Schicht. Allgemein sind hierdurch Wärme entwickelnde Schichten optimal kontaktierbar, welche nur über eine schlechte Oberflächenleitfähigkeit verfügen, wie allgemein spritzgegossene, elektrisch leitfähige Polymer-Compound-Bauteile. Eine solche Art der Kontaktierung bietet auch dann einen wesentlichen Vorteil, wenn aufgrund ungünstiger Oberflächenbeschaffenheit, zum Beispiel einer hohen Rauigkeit, eine anderweitige elektrische Kontaktierung in zuverlässiger und gleichmäßiger Weise nicht gewährleistet werden kann.

Nach einer weiteren Ausführungsform der Erfindung handelt es sich bei dem Heizelement um ein Pflaster. Insbesondere bei Pflastern ist es wünschenswert, dass eine hohe Beweglichkeit und gegebenenfalls auch Dehnbarkeit des Heizelements und/oder der flächigen, Wärme entwickelnden Schicht und damit der Kontaktelemente gewährleistet ist. Ein solches Pflaster kann direkt auf der Haut eines Benutzers Anwendung finden, wobei sich in diesem Fall die Oberflächentopographie des Heizelements mit einer Bewegung der Haut des Benutzers verändert.

Unter Pflastern im erfindungsgemäßen Sinn versteht der Fachmann flächige, auf der Haut selbstklebende und bei Bedarf wieder abziehbare Komposite, insbesondere mehrschichtig aufgebaute Flächengebilde, ganz besonders bevorzugt aufweisend mindestens eine Adhäsionsschicht und eine nicht klebende Trägerschicht. Pflaster können wirkstofffrei sein, aber sowohl in die Adhäsionsschicht als auch zwischen Adhäsions- und Trägerschicht, sowie innerhalb oder außerhalb der Trägerschicht können bei besonderen Ausführungsformen auch pharmazeutische oder kosmetische Wirkstoffe eingearbeitet sein, welche nach Applikation des Pflasters auf der Haut in die Haut diffundieren können (transdermales Pflaster). Diese Diffusionseffekte werden durch Wärmezufuhr aus dem Heizelement unterstützt bzw. verstärkt. In diesem Fall befindet sich das Heizelement auf jener Seite des Pflasters, welche von der Haut abgewandt ist, so dass die wirkstoffenthaltende Schicht Kontakt zur Haut haben kann.

In einem weiteren Aspekt betrifft die Erfindung ein Pflaster, umfassend ein Heizelement wie obig beschrieben.

Nach einer weiteren Ausführungsform der Erfindung ist das Pflaster selbstklebend.

Nach einer weiteren Ausführungsform der Erfindung weist das Pflaster einen pharmazeutischen oder kosmetischen Wirkstoff auf. Dieser Wirkstoff kann nach Applikation des Pflasters auf die Haut vom Pflaster an die Haut abgegeben werden. Das Heizelement kann, wie bereits oben beschrieben, diese Abgabe des Wirkstoffs beschleunigen.

In einem weiteren Aspekt betrifft die Erfindung ein Verfahren zur Herstellung eines Heizelements mit einer flächigen wärmeentwickelnden Schicht, wobei das Verfahren das Anordnen von zwei elektrischen Kontaktierungen an einander gegenüberliegenden Seiten der Schicht zur Bereitstellung eines elektrischen Heizstroms umfasst, wobei zumindest eine der Kontaktierungen ein erstes Kontaktelement und ein zweites Kontaktelement umfasst, wobei das Anordnen der zumindest einen Kontaktierung umfasst:
- Einbringen des ersten Kontaktelements in die wärmeentwickelnde Schicht in einer ersten Ebene senkrecht zur Fläche der Schicht gesehen, wobei sich das erste Kontaktelement in der wärmeentwickelnden Schicht in der ersten Ebene geschwungen erstreckt,
- Anbringen des zweiten Kontaktelements an der wärmeentwickelnden Schicht, wobei sich das zweite Kontaktelement parallel zur Erstreckungsrichtung des ersten Kontaktelements erstreckt, wobei das erste Kontaktelement und das zweite Kontaktelement in der Erstreckungsrichtung gesehen eine Vielzahl von gemeinsamen Kontaktpunkten aufweisen.

Es sei ferner angemerkt, dass die obig beschriebenen Ausführungsformen in beliebiger Weise miteinander kombiniert werden können, solange sich die kombinierten Ausführungsformen nicht gegenseitig ausschließen.

Im Folgenden werden bevorzugte Ausführungsformen der Erfindung anhand der Zeichnungen näher erläutert. Es zeigen:
- Figur 1: eine Ansicht eines Heizelements mit geschwungener erster Kontaktierung und geradliniger zweiter Kontaktierung,
- Figur 2: eine Ansicht des Heizelements der Figur 1, wobei jedoch hier auch für das zweite Kontaktelement eine geschwungene Form gewählt wurde,
- Figur 3: das Heizelement der Figur 2 im gedehnten Zustand,
- Figur 4: eine weitere Ansicht eines Heizelements mit geschwungener erster Kontaktierung,
- Figur 5: eine weitere Ansicht eines Heizelements mit geschwungener erster Kontaktierung.

Im Folgenden werden einander ähnliche Elemente mit den gleichen Bezugszeichen gekennzeichnet.

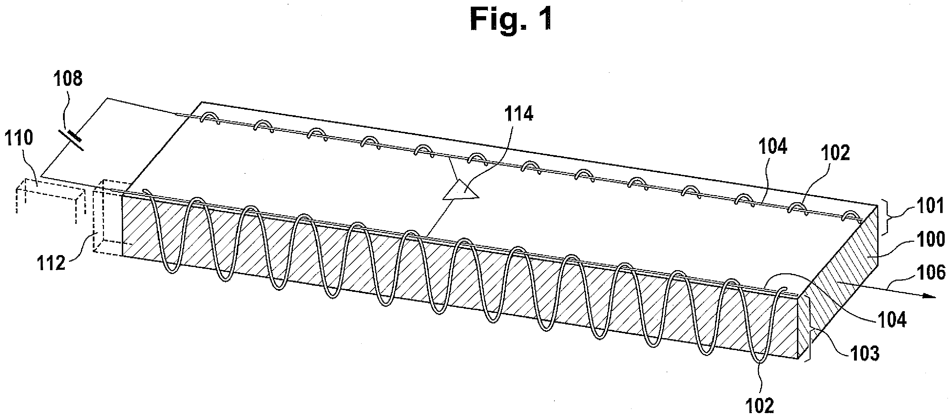

Die Figur 1 zeigt eine Schnittansicht eines Heizelements mit einer flächigen, Wärme entwickelnden Schicht 100. Diese Schicht weist eine gewisse Dicke auf, wobei in der Schicht (in deren Volumen) homogen ein elektrisch leitfähiges Material mit einem bestimmten elektrischen Widerstand verteilt ist. Es ist dabei durchaus möglich, dass dieser elektrische Widerstand je nach Schichttiefe variiert. Beispielsweise könnte der elektrische Widerstand an der Oberfläche sehr hoch sein, wohingegen der elektrische Widerstand in der Mitte der Schicht, das heißt auf halber Tiefe, geringer ist.

Zwei elektrische Kontaktierungen 101 und 103 dienen zur Bereitstellung eines elektrischen Heizstroms. Diese beiden elektrischen Kontaktierungen 101 und 103 sind dabei an der Wärme entwickelnden Schicht 100 an in paralleler Richtung zur Fläche der Schicht gesehen einander gegenüberliegenden Seiten der Schicht angeordnet. Ein Stromfluss kann also im Bild der Figur 1 quer von der elektrischen Kontaktierung 101 zur elektrischen Kontaktierung 103 fließen. Der dazwischenliegende Bereich der Wärme entwickelnden Schicht 100 wird sich dabei aufgrund seines elektrischen Widerstandes erwärmen.

Jede der elektrischen Kontaktierungen 101 und 103 umfasst nun ein erstes Kontaktelement 102 und ein zweites Kontaktelement 104. Das erste Kontaktelement 102 verläuft dabei geschwungen in der Wärme entwickelnden Schicht. Konkret erstreckt sich das Kontaktelement 102 in der Wärme entwickelnden Schicht in einer ersten Ebene, angedeutet durch die Querschnittsfläche 112 der Wärme entwickelnden Schicht, senkrecht zur Fläche der Schicht gesehen in geschwungener Form. Das erste Kontaktelement 102 taucht dabei regelmäßig von der Oberfläche der Wärme entwickelnden Schicht in das Volumen der Wärme entwickelnden Schicht hinein, durchdringt die Wärme entwickelnde Schicht, tritt aus der rückseitigen Oberfläche der Schicht wieder heraus, um dann wieder zurücklaufen zur anderen gegenüberliegenden Seite der Oberfläche. Im Beispiel der Figur 1 ist eine geschwungene Form gezeigt. Möglich sind jedoch auch Zickzackformen, Rechteckformen, Mäanderformen und vieles mehr.

Das zweite Kontaktelement 104 erstreckt sich parallel zur Erstreckungsrichtung 106 des ersten Kontaktelements 102. Dabei verläuft das zweite Kontaktelement 104 ebenfalls in einer Ebene, angedeutet durch die Fläche 110 der Wärme entwickelnden Schicht, welche sich parallel zur Oberfläche der Schicht 100 gesehen erstreckt. Da es sich im Beispiel der Figur 1 bei dem zweiten Kontaktelement 104 um einen länglichen, geraden Leiter, beispielsweise einen länglichen Draht, handelt, fallen für das zweite Kontaktelement die erste Ebene und die zweite Ebene zusammen.

Das zweite Kontaktelement 104 weist eine Vielzahl von gemeinsamen Kontaktpunkten mit dem ersten Kontaktelement 102 auf. Eine elektrische Kontaktierung des zweiten Kontaktelements 104 über eine Spannungsquelle 108 ermöglicht es also, Strom an einer Vielzahl von unterschiedlichen Kontaktpunkten in das erste Kontaktelement einzuspeisen. Selbst wenn also das erste Kontaktelement selbst einen nicht zu vernachlässigenden elektrischen Widerstand aufweist, ist dadurch dennoch gewährleistet, dass die Stromzufuhr über das erste Kontaktelement homogen über die gesamte Wärme entwickelnde Schicht, einschließlich deren inneren "Volumenbereich" gewährleistet ist.

Ferner ist gemäß dem Beispiel der Figur 1 ein Wärmesensor 114 vorgesehen, welcher in der Lage ist, über einen nicht näher gezeigten Schaltkreis die Stromzufuhr zu den elektrischen Kontaktierungen zu regeln. Beispielsweise kann über den Sensor 114 die erfasste Temperatur codiert und auf die zweiten Kontaktelemente aufmoduliert werden, sodass diese modulierte Information an einem nicht näher gezeigten Steuergerät dazu verwendet werden kann, die durch die Spannungsquelle 108 bereitgestellte Spannung bzw. den bereitgestellten elektrischen Strom zu steuern. Hierdurch kann eine gewünschte Temperatur einfach eingestellt werden.

Ohne der Beschränkung der Allgemeinheit wird im Folgenden davon ausgegangen, dass es sich bei dem ersten Kontaktelement um ein elektrisch leitfähiges Garn und bei dem zweiten Kontaktelement um einen metallischen Draht handelt. Ferner wird davon ausgegangen, dass die Wärme entwickelnde Schicht elektrisch leitfähige Nanopartikel, wie beispielsweise Nanoröhren oder Nanometallpartikel, enthält. Diese gewährleisten die elektrische Leitfähigkeit der Wärme entwickelnden Schicht. Es sei jedoch angemerkt, dass auch andere Materialien zum Einsatz kommen können, zum Beispiel gefüllte Polymerkomposite, welche elektrisch leitfähig sind, leitfähige Silikone oder elektrisch leitfähiges Papier, insbesondere elektrisch leitfähiges "Bucky-Paper". Letzteres beschreibt miteinander spagettiartig verschlungene Nanoröhren, welche aufgrund ihrer gegenseitigen Umschlingung ein stabiles und freistehendes elektrisch leitfähiges Material bilden.

Das erste Kontaktelement 102 in Form des elektrisch leitfähigen Garns kann beispielsweise ein metallbeschichtetes, z.B. Silber oder Gold beschichtetes Nylon umfassen, welches in Form eines Oberfadens in die Wärme entwickelnde Schicht eingenäht ist. Das zweite Kontaktelement 104 in Form beispielsweise einer Kupferlitze oder eines Kupferdrahtes ist dabei aufgrund seiner Aufbringung als Unterfaden automatisch mit dem Oberfaden, das heißt dem elektrisch leitfähigen Garn 102 verbunden. Durch das Hindurchstecken eines Garns durch die Wärme entwickelnde Schicht ergibt sich eine mechanisch stabile, formschlüssige Verbindung. Durch die zusätzliche Verwendung der im Vergleich zum elektrisch leitfähigen Garn 102 sehr hoch elektrisch leitfähigen Kupferlitze 104 ergibt sich damit eine gute mechanische Anbindung der Elektroden an die Wärme entwickelnde Schicht bei insgesamt geringen Eigenwiderständen der elektrischen Kontaktierungen 101 bzw. 103.

Durch die etwaige Flexibilität der verwendeten Materialien ist zudem gewährleistet, dass sich die Kontaktierung zusammen mit der Wärme entwickelnden Schicht verformen kann. Hierdurch erschließen sich flexible Anwendungsfelder für solche Heizelemente. Ein Beispiel für eine solche Anwendung ist die Verwendung direkt auf der Haut, welche ihre Oberflächentopographie mit einer Bewegung des Benutzers verändert.

Liegt insbesondere im Falle der Verwendung von Wärme entwickelnden Schichten, welche nur in ihrem Volumen elektrisch leitfähig sind, eine schlechte Oberflächenleitfähigkeit vor, liegt über die obig beschriebene Kontaktierung durch das elektrisch leitfähige Garn eine optimale und homogen verteilte Möglichkeit der Stromzufuhr über das gesamte Volumen der Wärme entwickelnden Schicht vor. Durch das Hindurchstecken des elektrisch leitfähigen Garns wird außerdem eine mechanisch stabile Anbindung des Garns 102 und über das Garn eine hochstabile Anbindung des Drahts 104 an die Wärme entwickelnden Schicht gewährleistet.

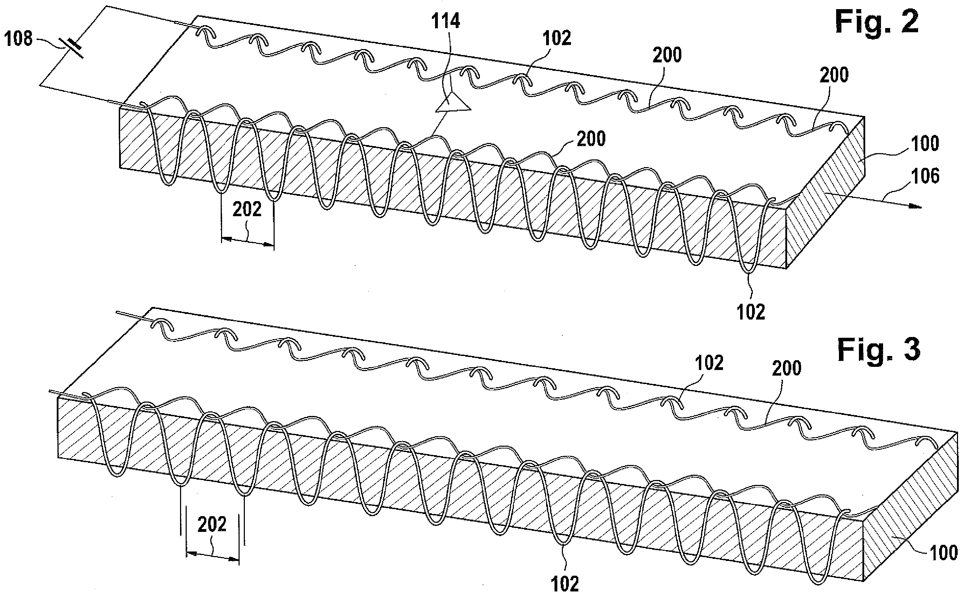

Die Figur 2 zeigt eine Variante des Beispiels der Figur 1, wobei hier der in Figur 1 gestreckte Draht 104 in der Figur 2 durch einen geschwungenen Draht 200 ersetzt wurde. Die Schwingung verläuft dabei in der Ebene, die mit dem Bezugszeichen 110 gekennzeichnet ist, das heißt parallel zur Oberfläche der Wärme entwickelnden Schicht 100. An der Oberfläche der Wärme entwickelnden Schicht bilden damit sowohl das erste Kontaktelement 102 als auch das zweite Kontaktelement 200 Schlaufen, welche ineinander eingreifen. Dadurch erfolgt eine indirekte Fixierung des zweiten Kontaktelements 200 an das erste Kontaktelement 102 und an die Wärme entwickelnde Schicht 100.

Das erste Kontaktelement 102 erstreckt sich in der ersten Ebene 112 mit einer räumlichen Schwingungsperiode, wobei eine Strecke einer solchen Periode in der Figur 2 durch das Bezugszeichen 202 gekennzeichnet ist. Das Bezugszeichen 202 bezeichnet also den räumlichen Abstand zwischen zwei Schlaufen des ersten Kontaktelements 102. Nun ist das erste Kontaktelement 102 dazu ausgebildet, im Falle einer Dehnbewegung der elektrisch leitfähigen Schicht 100 in Richtung 106 ebenfalls dieser Dehnbewegung zu folgen. Dies erfolgt aufgrund einer Änderung der Schwingungsperiode des ersten Kontaktelements.

Dies ist im Detail näher in Figur 3 erläutert, wo ersichtlich ist, dass nun der Abstand zwischen zwei Schlaufen nicht mehr dem Abstand 202, sondern einem größeren Abstand entspricht. Insgesamt wurde die elektrisch leitfähige Schicht in Richtung 106 gedehnt, das heißt verlängert. Als Konsequenz wurden die Schlaufen des ersten Kontaktelements 102 auseinandergezogen, was gegebenenfalls zu einer Straffung des ersten Kontaktelements 102 in der elektrisch leitfähigen Schicht führt. Ebenso wird das zweite Kontaktelement aufgrund seiner Schleifenform in der Lage sein, dieser Dehnbewegung in Richtung 106 in analoger Weise zur Änderung der Periode des ersten Kontaktelements zu folgen. Nichtsdestotrotz ist gewährleistet, dass eine gleichmäßige Stromzufuhr vom ersten Kontaktelement zu der Wärme entwickelnden Schicht über das gesamte Volumen der Schicht gesehen gewährleistet ist. Auch die mechanische Anbindung der verwendeten elektrischen Kontaktierungen leidet unter einen solchen Deformation des Heizelements nicht.

Abschließend sei angemerkt, dass zusätzlich zur geschwungenen Ausgestaltung des ersten und zweiten Kontaktelements dieses erste und zweite Kontaktelement auch selbst eine gewisse Eigenelastizität aufweisen kann. Dies kann durch eine geeignete Materialwahl gewährleistet werden und sichert zusätzlich eine hohe Dehnbarkeit und deren Reversibilität des gesamten Heizelements.

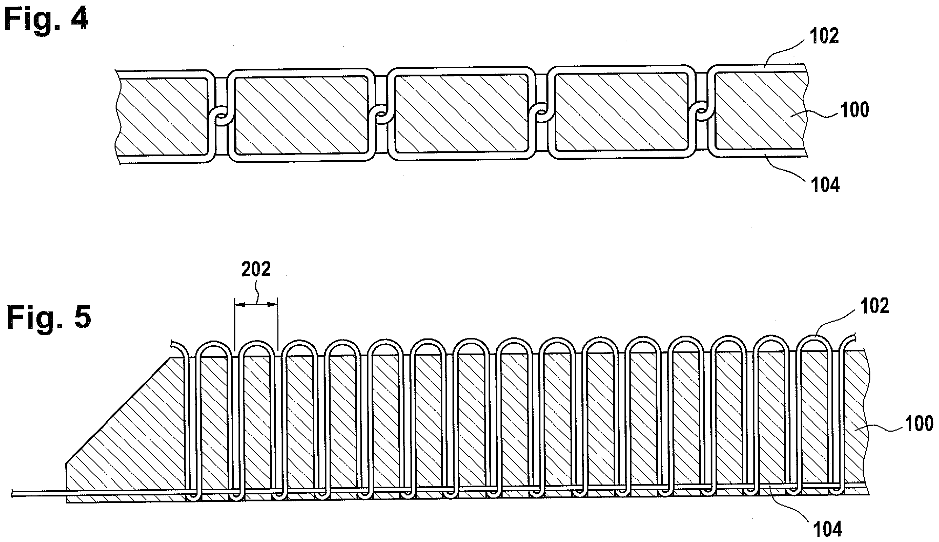

Die Figur 4 zeigt eine weitere Ansicht eines Heizelements mit geschwungener erster Kontaktierung. Im Unterschied zu den vorigen Ansichten werden hier das erste Kontaktelement 102 und das zweite Kontaktelement 104 durch das gleiche Loch in dem Heizelement zurückgeführt, wobei sich erstes und zweites Kontaktelement gegenseitig in dem Loch festhalten. Während also in den Figuren 1-3 das erste Kontaktelement 102 durch unterschiedliche Löcher auf der in diesen Figuren sichtbaren Oberfläche des Heizelements eingeführt und zurückgeführt werden, findet das Einführen und Rückführen in der Figur 4 durch das selbe Loch statt.
Eine Variante hierzu ist in der Figur 5 gezeigt, bei welcher im Gegensatz zur Figur 4 das zweite Kontaktelement 104 auf der Oberfläche des Heizelements 100 aufliegt und nicht mit in den Querschnitt gezogen wird. In den Figuren 4 und 5 bietet es sich an, dass das erste Kontaktelement einen Oberfaden und das zweite Kontaktelement einen Unterfaden bildet.

Da das erste Kontaktelement durch das selbe Einstichloch eingeführt und wieder ausgeführt wird, ergibt sich als die räumliche Periode 202 (vgl. Figur 2) der räumliche Abstand zwischen zwei benachbarten Einstichstellen.

### Bezugszeichenliste

- 100: Wärme entwickelnde Schicht
- 101: elektrische Kontaktierung
- 102: Kontaktelement
- 103: elektrische Kontaktierung
- 104: Kontaktelement
- 106: Dehnungsrichtung
- 108: Spannungsquelle
- 110: Ebene
- 112: Ebene
- 114: Sensor
- 200: Kontaktelement
- 202: Periode

## Patentansprüche

1. Heizelement mit einer flächigen wärmeentwickelnden Schicht (100) und zwei elektrischen Kontaktierungen (101; 103) zur Bereitstellung eines elektrischen Heizstroms, wobei die elektrischen Kontaktierungen (101; 103) an der wärmeentwickelnden Schicht (100) an einander gegenüberliegenden Seiten der Schicht (100) angeordnet sind, **dadurch gekennzeichnet, dass** zumindest eine der Kontaktierungen ein erstes Kontaktelement (102) und ein zweites Kontaktelement (104; 200) umfasst, wobei sich das erste Kontaktelement (102) in der wärmeentwickelnden Schicht (100) in einer ersten Ebene (112) senkrecht zur Fläche der Schicht (100) gesehen geschwungen erstreckt, wobei sich das zweite Kontaktelement (104; 200) parallel zur Erstreckungsrichtung (106) des ersten Kontaktelements erstreckt, wobei das erste Kontaktelement (102) und das zweite Kontaktelement (104; 200) in der Erstreckungsrichtung (106) gesehen eine Vielzahl von gemeinsamen Kontaktpunkten aufweisen.

2. Heizelement nach Anspruch 1, wobei sich das zweite Kontaktelement (104; 200) in einer zweiten Ebene (110) parallel zur Fläche der Schicht (100) gesehen erstreckt.

3. Heizelement nach Anspruch 2, wobei sich die zweite Ebene (110) an der Oberfläche der wärmeentwickelnden Schicht (100) befindet.

4. Heizelement nach einem der vorigen Ansprüche 2 oder 3, wobei sich das zweite Kontaktelement (104; 200) in der zweiten Ebene (110) geschwungen erstreckt.

5. Heizelement nach einem der vorigen Ansprüche, wobei das erste Kontaktelement (102) und/oder das zweite Kontaktelement (104; 200) in die wärmeentwickelnde Schicht (100) durch eine bei textilen Fügeverfahren verwendbare Verbindungstechnik eingebracht ist.

6. Heizelement nach Anspruch 5, wobei das erste Kontaktelement (102) und das zweite Kontaktelement (104; 200) durch ineinander hängenden Schlaufen die Vielzahl von gemeinsamen Kontaktpunkten aufweisen.

7. Heizelement nach einem der vorigen Ansprüche, wobei das erste Kontaktelement (102) die wärmeentwickelnde Schicht (100) in Richtung senkrecht zur Fläche der wärmeentwickelnden Schicht (100) vollständig durchdringt.

8. Heizelement nach einem der vorigen Ansprüche, wobei das erste Kontaktelement (102) ein elektrisch leitfähiges Garn umfasst.

9. Heizelement nach einem der vorigen Ansprüche, wobei es sich bei dem ersten Kontaktelement um einen Oberfaden und bei dem zweiten Kontaktelement um einen Unterfaden handelt.

10. Heizelement nach einem der vorigen Ansprüche, wobei die wärmeentwickelnde Schicht (100) aufgrund einer Dehnbewegung elastisch dehnbar ist, wobei sich das erste Kontaktelement (102) in der ersten Ebene mit einer Schwingungsperiode geschwungen erstreckt, wobei das erste Kontaktelement (102) dazu ausgebildet ist, aufgrund einer Änderung der Schwingungsperiode der elastischen Dehnbewegung zu folgen.

11. Heizelement nach einem der vorigen Ansprüche, wobei die flächige wärmeentwickelnde Schicht (100) homogen verteilt
- elektrisch leitfähige Nanopartikel, insbesondere Nanoröhren und/oder
- ein elektrisch leitfähiges Polymer aufweist.

12. Pflaster, umfassend ein Heizelement nach einem der vorigen Ansprüche.

13. Pflaster nach Anspruch 12, wobei das Pflaster selbstklebend ist.

14. Pflaster nach Anspruch 12 oder 13, wobei das Pflaster einen pharmazeutischen oder kosmetischen Wirkstoff aufweist.

15. Verfahren zur Herstellung eines Heizelements mit einer flächigen wärmeentwickelnden Schicht (100), wobei das Verfahren das Anordnen von zwei elektrischen Kontaktierungen (101; 103) an einander gegenüberliegenden Seiten der Schicht (100) zur Bereitstellung eines elektrischen Heizstroms umfasst, wobei zumindest eine der Kontaktierungen ein erstes Kontaktelement (102) und ein zweites Kontaktelement (104; 200) umfasst, wobei das Anordnen der zumindest einen Kontaktierung umfasst:
- Einbringen des ersten Kontaktelements (102) in die wärmeentwickelnde Schicht (100) in einer ersten Ebene (112) senkrecht zur Fläche der Schicht (100) gesehen, wobei sich das erste Kontaktelement (102) in der wärmeentwickelnden Schicht (100) in der ersten Ebene (112) geschwungen erstreckt,
- Anbringen des zweiten Kontaktelements (104; 200) an der wärmeentwickelnden Schicht (100), wobei sich das zweite Kontaktelement (104; 200) parallel zur Erstreckungsrichtung (106) des ersten Kontaktelements erstreckt, wobei das erste Kontaktelement (102) und das zweite Kontaktelement (104; 200) in der Erstreckungsrichtung (106) gesehen eine Vielzahl von gemeinsamen Kontaktpunkten aufweisen.

## Claims

1. A heating element, comprising a planar heat-generating layer (100) and two electrical contacts (101; 103) for providing an electric heating current, wherein the electrical contacts (101; 103) are arranged on the heat-generating layer (100) on mutually opposed sides of the layer (100), **characterised in that** at least one of the contacts comprises a first contact element (102) and a second contact element (104; 200), wherein the first contact element (102) extends in the heat-generating layer (100) in an undulating manner as viewed in a first plane (112) perpendicular to the extent of the layer (100), wherein the second contact element (104; 200) extends parallel to the extension direction (106) of the first contact element, wherein the first contact element (102) and the second contact element (104; 200) have a multiplicity of common contact points as viewed in the extension direction (106).

2. The heating element according to claim 1, wherein the second contact element (104; 200) extends in a second plane (110) parallel to the extent of the layer (100).

3. The heating element according to claim 2, wherein the second plane (110) is disposed on the surface of the heat-generating layer (100).

4. The heating element according to either one of the preceding claims 2 or 3, wherein the second contact element (104; 200) extends in an undulating manner in the second plane (110).

5. The heating element according to any one of the preceding claims, wherein the first contact element (102) and/or the second contact element (104; 200) are/is introduced into the heat-generating layer (100) by means of a connection technique usable in textile joining methods.

6. The heating element according to claim 5, wherein the first contact element (102) and the second contact element (104; 200) have the multiplicity of common contact points due to loops looped one inside the other.

7. The heating element according to any one of the preceding claims, wherein the first contact element (102) fully penetrates the heat-generating layer (100) in the direction perpendicular to the extent of the heat-generating layer (100).

8. The heating element according to any one of the preceding claims, wherein the first contact element (102) comprises an electrically conductive thread.

9. The heating element according to any one of the preceding claims, wherein the first contact element is an upper thread and the second contact element is a lower thread.

10. The heating element according to any one of the preceding claims, wherein the heat-generating layer (100) is resiliently extendible on account of a stretching movement, wherein the first contact element (102) extends in an undulating manner in the first plane with an oscillation period, wherein the first contact element (102) is configured to follow the elastic stretching movement on account of a change to the oscillation period.

11. The heating element according to any one of the preceding claims, wherein the planar heat-generating layer (100) comprises, in a homogeneously distributed manner,
- electrically conductive nanoparticles, in particular nanotubes and/or
- an electrically conductive polymer.

12. A compress, comprising a heating element according to any one of the preceding claims.

13. The compress according to claim 12, wherein the compress is self-adhesive.

14. The compress according to claim 12 or 13, wherein the compress comprises a pharmaceutical or cosmetic active substance.

15. A method for producing a heating element comprising a planar heat-generating layer (100), wherein the method comprises arranging two electrical contacts (101; 103) on mutually opposed sides of the layer (100) in order to provide an electric heating current, wherein at least one of the contacts comprises a first contact element (102) and a second contact element (104; 200), wherein the arranging of the at least one contact comprises:
- introducing the first contact element (102) into the heat-generating layer (100) in a first plane (112) as viewed perpendicular to the extent of the layer (100), wherein the first contact element (102) extends in the heat-generating layer (100) in an undulating manner in the first plane (112),
- applying the second contact element (104; 200) to the heat-generating layer (100), wherein the second contact element (104; 200) extends parallel to the extension direction (106) of the first contact element, wherein the first contact element (102) and the second contact element (104; 200) have a multiplicity of common contact points as viewed in the extension direction (106).

## Revendications

1. Elément de chauffage avec une couche génératrice de chaleur (100) plane et deux connexions électriques (101 ; 103) pour la mise en place d'un courant électrique de chauffage, où les connexions électriques (101 ; 103) sont disposées sur des côtés opposés l'un à l'autre sur la couche génératrice de chaleur (100), **caractérisé en ce qu'**au moins un des connexions comprend un premier élément de contact (102) et un deuxième élément de contact (104 ; 200), où le premier élément de contact (102) s'étend courbé dans la couche génératrice de chaleur (100) dans un premier plan (112), vu perpendiculairement par rapport à la surface de la couche (100), où le deuxième élément de contact (104 ; 200) s'étend parallèlement à la direction d'extension (106) du premier élément de contact, où le premier élément de contact (102) et le deuxième élément de contact (104 ; 200) présentent une multitude de points de contact communs, vu dans la direction de l'extension (106).

2. Elément de chauffage selon la revendication 1, dans lequel le deuxième élément de contact (104 ; 200) s'étend dans un deuxième plan (110), vu de manière parallèle par rapport à la surface de la couche (100).

3. Elément de chauffage selon la revendication 2, dans lequel le deuxième plan (110) se situe à la surface de la couche génératrice de chaleur (100).

4. Elément de chauffage selon l'une des revendications précédentes 2 ou 3, dans lequel le deuxième élément de contact ((104 ; 200) s'étend en se balançant dans le deuxième plan (110).

5. Elément de chauffage selon l'une des revendications précédentes, dans lequel le premier élément de contact (102) et/ou le deuxième élément de contact (104 ; 200) est (sont) logé(s) dans la couche génératrice de chaleur (100) par une technique de liaison pouvant être utilisée dans un procédé d'assemblage textile.

6. Elément de chauffage selon la revendication 5, dans lequel le premier élément de contact (102) et le deuxième élément de contact (104 ; 200) présentent la multitude des points de contact communs par des boucles pendantes entremêlées.

7. Elément de chauffage selon l'une des revendications précédentes, dans lequel le premier élément de contact (102) traverse totalement la couche génératrice de chaleur (100) dans une direction perpendiculaire à la surface de la couche génératrice de chaleur (100).

8. Elément de chauffage selon l'une des revendications précédentes, dans lequel le premier élément de contact (102) comprend un fil électriquement conducteur.

9. Elément de chauffage selon l'une des revendications précédentes, dans lequel il s'agit d'un fil supérieur dans le cas du premier élément de contact et d'un fil inférieur dans le cas du deuxième élément de contact.

10. Elément de chauffage selon l'une des revendications précédentes, dans lequel la couche génératrice de chaleur (100) est extensible élastiquement du fait d'un mouvement d'extension, où le premier élément de contact (102) s'étend dans le premier plan, se balançant avec une période d'oscillation, où le premier élément de contact (102) est conçu pour accompagner le mouvement d'extension élastique du fait de la modification de la période d'oscillation.

11. Elément de chauffage selon l'une des revendications précédentes, dans lequel la couche génératrice de chaleur (100) présente
- des nanoparticules électriquement conductrices, notamment des nanotubes, distribués de manière homogène, et/ou
- un polymère électriquement conducteur.

12. Pansement, comprenant un élément de chauffage selon l'une des revendications précédentes.

13. Pansement selon la revendication 12, où le pansement est autocollant.

14. Pansement selon la revendication 12 ou 13, où le pansement présente une matière active pharmaceutique ou cosmétique.

15. Procédé de fabrication d'un élément de chauffage avec une couche génératrice de chaleur (100) plane, où le procédé comprend l'agencement de deux connexions électriques (101 ; 103) sur des côtés opposés l'un à l'autre de la couche (100) pour la mise en place d'un courant électrique de chauffage, où au moins une des connexion comprend un premier élément de contact (102) et un deuxième élément de contact (104 ; 200), où l'agencement de l'au moins une connexion comprend :
- l'incorporation du premier élément de contact (102) dans la couche génératrice de chaleur (100) dans un premier plan (112), vu perpendiculairement à la surface de la couche (100), où le premier élément de contact (102) s'étend dans la couche génératrice de chaleur (100), se balançant dans le premier plan (112),
- la mise en place du deuxième élément de contact (104 ; 200) sur la couche génératrice de chaleur (100), où le deuxième élément de contact (104 ; 200) s'étend parallèle à la direction d'extension (106) du premier élément de contact, où le premier élément de contact (102) et le deuxième élément de contact (104 ; 200) présentent une multitude de points de contact communs, vu dans la direction de l'extension (106).
